# EUROPEAN PATENT APPLICATION

(11) **EP 0 536 454 A1**
(43) Date of publication of application: **14.04.1993**
(21) Application number: 91309369.6
(22) Date of filing: 10.10.1991
(51) Int. Cl.: A61F 2/16

(54) **Assembly method for intraocular lenses**

(71) Applicant: NESTLE S.A., CH-1800 Vevey (CH)
(72) Inventor: Peterson,S.C., c/o Minnesota Min.and Manuf.Co., St. Paul, Minnesota 55133-3427 (US)
(74) Representative: Baillie, Iain Cameron

(57) **Abstract**

A method for joining attachment members (haptics) to an intraocular lens (10) by employing a low enery laser. The haptics (40a),(40b) are used to secure the lens to the human eye. The filament ends of the haptics are first inserted into micro channels (65a),(65b) within the lens. A beam of laser light (70) is focused onto a portion of the filament within the lens channel. The material of the filament is such that the laser light beam is selectively absorbed by the filament causing it to melt and become joined to the lens body (30) without heating the lens body all along the path of the laser beam through the lens. The low energy laser beam does not cause penetration marks, holes, or other depressions on the surface of the lens at the entry point of contact of the laser beam with the lens surface. The novel method permits fixation of the filament to the lens body without physically contacting the lens body.

## Description

### Technical Field

The invention relates to intraocular lenses for insertion into the human eye. The invention particularly relates to a method of attaching haptics to an intraocular lens optic using low energy lasers.

### Background Art

Intraocular lenses are typically employed in cataract surgery to replace natural lenses. In the early 1970's a variety of intraocular lenses were developed having metal loops attached to the lens by drilling holes through the lens body and inserting the metal loops through the holes. The metal loops were bent to help keep them in place in the lens. Thermoplastic loops were also employed by this time. These plastic loops, conventionally referred to as plastic haptics, were typically attached by press-fitting filaments of the plastic material through predrilled holes having diameter smaller than the diameter of the plastic filament. Alternatively the plastic loops were attached by drilling holes entirely through the lens body and inserting the loop line through the hole. The end of the line was soldered or otherwise heated to cause a beading at the end of the line and the beading end was pushed back into the hole. In the latter 1970's method became available to permit direct contact heating of the lens body by a heating probe to help secure or fuse the loop attachment to the lens without adversely affecting the optical quality of the lens in the vision area.

In U.S. Patent No. 4,104,339, a method is disclosed for fusing the loop ends to the lens body by melting of the lens body. In the method described in this reference, holes are fist drilled into the side walls of the lens body. The holes are drilled typically laterally from the side edge into a portion of the lens body. The ends of a plastic loop are inserted into these holes. The lens body was typically of polymethylmethacrylate and the loop was typically a theremoplastic sold under the trade name "Supramid". A fused connection of the plastic loop ends to the lens body is formed by pushing an inductively heated probe through the lens body into contact with the thermoplastic loop material. The probe is designed to heat the lens material to a temperature slightly above the melting point of the lens body material. A temperature of about 190°F is taught as sufficient to cause melting of the polymethylmethacrylate lens material.

In U.S. Patent No. 3,994,027, a prepupillary lens is disclosed for implantation in the human eye. The lens is commonly of polymethylmethacrylate (PMMA) and has a planar surface and a convex surface. Drilling of microscopic holes to secure the posterior loops to the lens body is eliminated. The posterior loops are formed from platinum-iridium wire. The wires are heated to a temperature between about 125°C to 200°C. the heated wire prong ends are inserted into the planar surface of the lens and perpendicular thereto. When the heated prongs have cooled they are fused to the lens. Heated wire or heated probe staking methods involve complicated set up and cause penetration holes on the surface of the lens. Cellular or other fibrous debris can collect over time in such penetration holes and result in eye discomfort.

In U.S. Patent No. 4,786,445 a process employing an ND:YAG laser for securing filaments to an intraocular lens is disclosed. In the process disclosed the filament channel has been modified by providing the channel with a shoulder or else providing the channel with an irregularity such as internal threading. As laser energy is focused onto a portion of the filament within the channel it causes the filament portion to melt. The melted filament portion expands into the locking shoulder or other irregularity provided within the channel. As the melted portion of the filament expands into the shoulder or other irregularity in the channel, a "mechanical interlock" is created. The ND:YAG laser employed uses neodymium in a matrix of yttrium-aluminum garnet. Such lasers are high energy lasers typically operating at powers of 25 to 50 watts. This laser generates radiant energy in the near infrared range having a wavelength of between 1060 and 1300 nanometers. Since the ND:YAG laser is a high energy laser, its use is inherently dangerous and operational control is complicated.

### Disclosure of Invention

The invention in its principal aspect is a method employing laser energy to affix attachment members, such as filaments or loops, known on the art as "haptics", to an intraocular lens intended for implant in the human eye. The attachment members may be of conventional shape and are employed to hold the lens securely in the human eye. Micro channels or holes are first drilled into the intraocular lens body in conventional manner. At least one end of the attachment member is inserted into the channels. A beam of low level laser energy of less than about 1 watt and having a wave length preferably in the visible light spectrum is focused onto a portion of the attachment member in the micro channel within the lens. Material for the attachment member is selected in combination with a laser source which generates laser energy at the proper wavelength such that energy of the laser beam is preferentially absorbed by the attachment member rather than the lens material. The attachment member absorbs the laser energy, and thereby becomes heated sufficiently to soften or melt. After cooling, the attachment member becomes instantly and permanently affixed to the inside walls of the channel within the lens body.

An important advantage of the laser process of the present invention is that the laser beam is of low energy, less than about 1 watt, thereby reducing the risk of injury to operators or surroundings. Additionally, the laser process of the present invention does not create penetration marks or other depressions in the surface of the lens body as it passes through the lens body to a portion of the attachment member being held in the micro channel. Another important advantage is that there is no chance of dislodging the filament within the channel immediately prior to or during the affixing process, since the laser beam does not cause movement of the lens body as it heats the attachment member filament. This is a distinct advantage over conventional methods of attaching haptics to the lens bodies by employing a heated probe which physically contacts the lens body.

The lens body and attachment member may be of the same or different materials, so long as at least a portion of the attachment member which resides in the lens body and would be in the path of the laser beam is capable of selectively absorbing sufficient low level laser energy to soften or melt, while the lens body does not absorb sufficient laser energy to cause appreciable melting. Preferably a pigment or dye is used to enable the attachment member to selectively absorb the laser energy.

The attachment member and lens body are advantageously of the same material preferably polymethylmethacrylate. In this case the attachment member or filament is preferably colored during extrusion with a blue pigment or violet dye. Where the pigment is blue, the laser source is preferably a helium neon laser. Where the dye is violet, the laser source is preferably an argon or helium neon laser. The laser energy is absorbed by the colored attachment member, thus heating the attachment member, but is not absorbed in the uncolored clear polymethylmethacrylate lens body. The attachment member may be colored with other pigments or dyes. In such case a laser source could be selected which generates laser energy at a wavelength which is selectively absorbed and converted to thermal energy by the filament without directly heating the lens body. The lens body and attachment member may be of different materials. If so they are both advantageously thermoplastic with the attachment material preferably having nearly the same or lower softening and melting point than the lens body material.

### Brief Description of the Drawings

FIG. 1 is a schematic representation showing the intraocular lens with filament positioned therein and laser source in relation to the lens.

FIG. 2 is a schematic representation as in FIG. 1 with the base of the lens and filament enlarged.

### Detailed Description

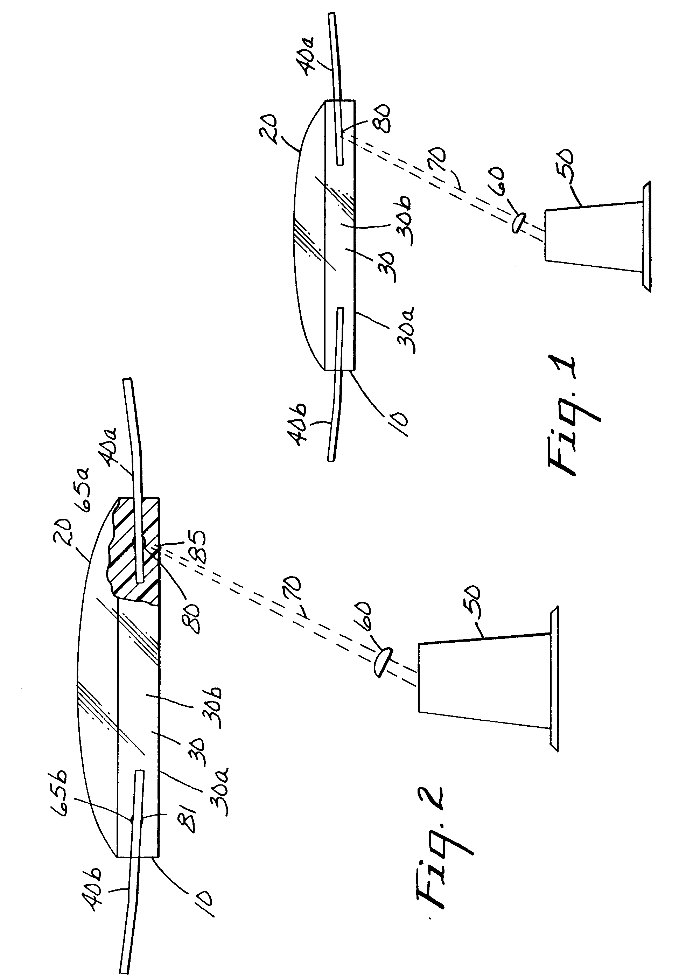

A preferred embodiment illustrating the invention is shown in FIG. 1 and FIG. 2. An intraocular lens 10 as illustrated in the figures is composed of a lens body 30 and identical attachment member filaments 40a and 40b. The lens body 30 is an optical element having a convex anterior surface 20, a posterior planar surface 30(a) and a cylindrical peripheral surface 30(b). Attachment member filaments 40a and 40b are single strand filaments. Channels are microdrilled into the lens body 30 preferably from opposing sides in peripheral surface 30b, thus forming channels 65a and 65b. Such channels are preferably straight holes of cylindrical shape and uniform diameter along their length. Channels 65a and 65b typically have a diameter of about 0.15 mm. The ends of filaments 40a and 40b are inserted respectively into channels 65a and 65b. Such filament has a diameter which is preferably just slightly less than the diameter of the channel into which it is inserted. After the channels are microdrilled into peripheral surface 30b, the lens body 30 is preferably placed on a holder (not shown) and secured thereto by applying a vacuum between the posterior surface 30a of the lens body 30 and the holder. The ends of the filaments 40a and 40b are manually inserted into channels 65a and 65b respectively, advantageously using a microscope as a guide and tweezers to grasp and insert the filament. After the ends of the filaments 40a and 40b are inserted into the channels 65a and 65b, an open cap (not shown) is secured over the lens to hold the filaments in place and then the vacuum is released. Filaments 40a and 40b are then joined permanently to the lens body 30 by directing a beam of low energy laser light of less than about 1 watt typically between 10 milliwatt and 1 watt power to a point preferably near the middle portion of each filament 40a and 40b which lies in channels 65a and 65b, respectively. The low energy laser can be readily focused with pinpoint accuracy. It was surprising that low level laser energy of less than about 1 watt, preferably from 10 to 900 milliwatts and most preferably between 10 and 500 milliwatts, could be employed to affix filaments 40a and 40b to lens body 30 within respective channels 65a and 65b. The low level laser energy permanently affixed filaments 40a and 40b to the lens without causing melting or softening of the lens body 30 at the point of entry of the laser beam into the lens body. The filament 40a and 40b becomes permanently affixed in channels 65a and 65b, respectively without any need for mechanical interlocking mechanism. Filaments 40a and 40b become firmly affixed to lens body 30 instantly upon cooling of the filaments under ambient conditions immediately after the filaments have been contacted by the low level laser energy. Once the filaments 40a and 40b become fixed to lens body 30 they will not dislodge from their respective channels 65a and 65b.

Since the laser beam 70 emanating from laser apparatus 50 does not melt or soften the lens body at the point of entry 85 into lens 30, there are no indentation marks or other surface depressions or pin holes caused by laser beam 70 in the surface of the lens 30. There may occasionally occur a microscopic welt of height less than about 0.02 mm which is very significantly less than the filament diameter. The filament diameter is typically 0.15 mm. If such a welt occurs it is caused by the heat from the filament and not by the laser beam. The welt is of no consequence should it occur since it does not break the surface of the lens and is outside the optical area.

The lens body 30 is constructed of material which will transmit the low level laser energy therethrough without absorbing any of the laser energy and without causing significant melting of the lens body. Preferably the lens body 30 is constructed of polymethylmethacrylate. The attachment members 40a and 40b are constructed at least in part of a material which absorbs sufficient laser energy transmitted through the lens body to become softened and molten. The attachment members 40a and 40b may be composed of a material preferably having the same or lower softening and melting point than lens body 30. A preferred material from which to construct filaments 40a and 40b is polymethylmethacrylate colored with a blue pigment such as copper-phthalocyanine, or a violet dye such as D and C Violet #2. Applicant has discovered that if the attachment members, e.g. filaments 40a and 40b, are properly colored the low energy laser source 50 will cause softening and melting only of the filaments, without causing significant softening or melting of the lens body, even though the lens body and attachment member are constructed of the same material, e.g. polymethylmethacrylate.

The preferred laser source 50 is a helium neon gas or argon laser. The helium neon laser for use in the present process is preferably a continuous output laser which has a power between about 10 and 50 milliwatts, preferably 35 milliwatts and a wavelength of 632.8 nm. A suitable helium neon laser source 50 may be purchased from Spectra Physics Co., Mountain View, California. The argon laser for use in the present process is preferably a continuous output laser which has a power between about 85 and 115 milliwatts, preferably 100 milliwatts and major wavelengths of 488 and 514 nm. A suitable argon laser source 50 is commercially available as a Model 2000 Argon Laser from Spectra-Physics Co. The laser source 50 may suitably be placed a few feet away from the filament 40, but it should be appreciated that this distance is not critical. The invention is not intended to be limited to a helium neon or argon lasers, since other low energy laser sources operating at less than 1 watt beam power could also be employed. Other low energy lasers of less than 1 watt beam power and preferably having a wavelength of between 400 to 1000 nanometers are also possible. For example, a tunable dye laser with beam power adjusted to less than 1 watt and wavelength between 400 to 1000 nanometers could also be employed. The laser source 50 is fitted with conventional focusing lenses, such as lens 60, in order to properly focus the laser beam 70 to the target contact point 80 on filament 40a. The preferred helium neon laser generates 633 nanometer visible red light beam. This wavelength is readily absorbed by the blue copper phthalocyanine pigmented polymethylmethacrylate material. The preferred argon laser generates 488 and 514 nanometer visible blue-green light. This wavelength is readily absorbed by the violet (D and C Violet #2) pigmented polymethylmethacrylate material. Other pigments or dyes of different color could be utilized in the filament to provide selective heating of the filament by a laser beam. If different pigments or dyes are chosen, then a low energy laser source of a different wavelength may be required in order to optimize the absorption of laser energy into the pigmented thermoplastic filament 40a without directly heating the lens material. Laser energy of continuous output at less than 1 watt for example between 10 milliwatts and 1 watt and at wavelengths of preferably between about 400 to 1000 nanometers could be employed provided a suitable absorbing pigment is chosen for filament 40a so that the filament absorbs sufficient laser energy at this wavelength to soften or melt. Thus, the present invention is not intended to be limited to one source of laser energy, nor to one color or type of pigment for the attachment members nor to one type or color of material for the optic body. Having made the present disclosure it is possible for those skilled in the art to find other pigments or dyes of colors other than blue copper phthalocyanine and D and C Violet #2, for example, for use in filament 40a. If another pigment is chosen it is possible to select a low energy laser energy source which generates light at the proper wavelength so that the light will be selectively absorbed by the filament without causing absorption in the lens body itself.

The present invention has significant advantages over prior art methods. Since the present laser process does not disturb the surface of the lens by causing penetration marks, holes, or other depressions in the lens surface, there is no chance of buildup of cellular and fibrous debris at such points. Prior art methods which employ a heated probe leave holes or depressions in the surface of the lens at the point of contact of such probes on the lens surface. Cellular and fibrous debris may accumulate in such holes or surface disturbances over time, especially when the surface inside the depression is rough. This in turn may lead to eye inflammation and discomfort. The process of the present invention clearly eliminates such a risk. Also, since the laser method of the present invention does not physically cause movement of the lens as the laser passes through the lens, there is no chance that the filament will become dislodged during the actual joining of filament to lens. The present invention is also safer and easier to operate than prior art methods employing high energy lasers.

### Example 1

A clear 6.5 mm diameter biconvex polymethacrylate lens was first prepared by drilling one straight cylindrical microchannel into the lens on each side thereof. Each channel was 0.15 mm in diameter and was drilled to a depth of about 1 mm with a high speed steel drill. Each channel was intended to accept one filament in open loop configuration. The lens was placed in a holder and was secured to the holder by applying a vacuum between the posterior surface of the lens and the holder. One filament of polymethylmethacrylate pigmented with blue copper phthalocyanine and having a diameter of about 0.145 mm was inserted into one hole and another filament of the same type was inserted into the other hole on the opposite side of the lens. Each filament was inserted manually using tweezers with the visual aid of a microscope. An open cap of about 1 inch in diameter was then secured over the lens to hold the filaments in place and then the vacuum was released. A beam of laser energy from a helium neon laser operating at a continuous power of about 35 milliwatts at 633 nonometers wavelength was focused onto one of the filaments at a point approximately midway along the length of the filament portion within its channel. The laser source was model 127-35 Stabilite helium neon laser made by Spectra Physics. The laser source was located about 2 feet distance from the filament. An available focusing lens of 1 inch diameter and focal length of 150 mm was employed to focus the laser beam onto the filament. The laser beam was focused onto the filament for a period sufficient to melt at least a portion of the filament within the channel as seen with the aid of a microscope. The filament was exposed to the laser beam for a period of about 5 seconds. The same process was then repeated by focusing the laser beam onto a second filament at a point about midway along the filament portion within the second channel. The open cap holding the filaments in place was removed. There were no discernible depression or holes made on the lens surface by the laser beam even when the lens surface was viewed under magnification of 10 to 30X. The bond between the filament and lens was found to be strong enough to allow a pull force of at least 50 gms to be applied to the filament without dislodging the filament from the lens channel. Under visual inspection using a high power 30X microscope there was no evidence of melting the lens along the path of the laser from its entry point into the lens up to the immediate vicinity of contact between laser beam and filament. It was found by separate experiments that the helium neon laser at low power levels of 35 milliwatts did not cause any melting of the lens even if the beam was focused onto the lens for long periods of time for example extending beyond even 10 minutes.

### Example 2

A clear 6.5 mm diameter biconvex polymethacrylate lens was first prepared by drilling one straight cylindrical microchannel into the lens on each side thereof. Each channel was 0.15 mm in diameter and was drilled to a depth of about 1 mm with a high speed steel drill. Each channel was intended to accept one filament in open loop configuration. The lens was placed in a holder and was secured to the holder by applying a vacuum between the posterior surface of the lens and the holder. One filament of polymethylmethacrylate pigmented with D&C violet #2 dye throughout and having a diameter of about 0.145 mm was inserted into one hole and another filament of the same type was inserted into the other hole on the opposite side of the lens. Each filament was inserted manually using tweezers with the visual aid of a microscope. A beam of laser energy from an argon laser operating at a continuous power of about 100 milliwatts at 488 and 514 nanometers wavelength was focused onto one of the filaments at a point approximately midway along the length of the filament portion within its channel. The laser source was model 2000 Argon Laser made by Spectra Physics. The laser source was located about 2 feet distance from the filament. An available focusing lens of 1 inch diameter and focal length of 100 mm was employed to focus the laser beam onto the filament. The laser beam was focused onto the filament for a period sufficient to melt at least a portion of the filament within the channel as seen with the aid of a microscope. The filament was exposed to the laser beam for a period of about 2 seconds. The same process was then repeated by focusing the laser beam onto a second filament at a point about midway along the filament portion within the second channel. There were no discernible depression or holes made on the lens surface by the laser beam even when the lens surface was viewed under magnification of 10 to 30X. Under visual inspection using a high power 30X microscope there was no evidence of melting the lens along the path of the laser from its entry point into the lens up to the immediate vicinity of contact between laser beam and filament.

It should be appreciated that the present process of the invention is not limited to the configuration or number of attachment members that are employed. The laser process of the present invention is also not limited to any shape of lens employed. Also, it should be appreciated that the process of the present invention is not limited to any particular position of the attaching holes or channels made in the lens for receiving the filaments. For example, the attaching channels 65a and 65b could be made in the posterior planar surface 30a of the lens instead of the sides presently illustrated in FIG. 1. Also, any conventional filament loop configuration, for example, including those illustrated in U. S. Patent Nos. 4,104,339 and 4,578,078 could be used with the present laser process. It will be apparent that any number of filaments can be joined in turn by the present process. For example, after joining of filament 40a to lens body 30 at point 80, laser beam 70 may be refocused through lens 60 or equivalent lens such that beam 70 is directed to a similar target contact point 81 on filament 40b. It should be appreciated that other materials or other low energy laser sources may be substituted for the specific materials and energy sources described in the preferred embodiments herein. The invention is thus not intended to be limited to the specific preferred embodiments but rather the scope of the invention is defined by the claims and equivalents thereof.

## Claims

1. A process for joining an attachment member to a lens body to produce an intraocular lens for implantation into the human eye wherein a channel is formed in at least a portion of the lens body and said attachment member inserted therein, characterized in that the process comprises:
joining said attachment member to the lens body by directing a laser energy beam of less than 1 watt through at least a portion of the lens body and onto at least a portion of the attachment member in said channel resulting in sufficient heating of said attachment member to join the attachment member to the lens body, wherein the attachment member within said channel has the property that it absorbs laser energy on contact therewith to become sufficiently soft or molten to become affixed within said channel in said lens body, and said lens body at least in the portion which is in the path of the laser beam has the property that it does not absorb the laser beam energy in sufficient amount to cause discernible penetration marks or depressions on the surface of the lens body.

2. A process as in Claim 1 wherein at least the portion of the lens body in the path of the laser beam is a different color from the attachment member in the path of said beam.

3. A process as in Claim 1 wherein the laser energy beam operates at a power level of between about 10 and 900 milliwatts.

4. A process as in Claim 2 wherein the attachment portion in the path of the laser beam is visibly violet in color and the laser energy beam is an argon or helium neon laser.

5. A process as in Claim 2 wherein the attachment portion in the path of the laser beam is visibly blue in color and the laser energy is a helium neon laser.
